# EUROPEAN PATENT SPECIFICATION

(11) **EP 1 417 969 B1**
(45) Date of publication and mention of the grant of the patent: **06.09.2006**
(21) Application number: 03024296.0
(22) Date of filing: 23.10.2003
(51) Int. Cl.: A61K 35/74, A61K 31/715, A61P 13/08

(54) **A pharmaceutical composition for the medical treatment of the benign prostatic hyperplasia, its preparation method and its therapeutical application**
Pharmazeutische Zusammentsetzung zur medizinischen Behandlung von gutartiger prostathyperplasie, dessen Vorbereitungsmethode und dessen therapeutische Verwendung
Composition pharmaceutique pour le traitement de l'hyperplasie prostatique bénigne, sa méthode de préparation et son application thérapeutique

(30) Priority: 08.11.2002 AR 0104293
(43) Date of publication of application: 12.05.2004
(73) Proprietor: H.P.B. Sociedad Anonima, 1187 BUENOS AIRES (AR)
(72) Inventor: Barreiro, Hipolito Carmelo Maria, 1187 Buenos Aires (AR)
(74) Representative: Barlocci, Anna

(56) References cited:
- EP-A- 0 295 961
- WO-A-02/052955
- LICHIUS J J ET AL: "Antiproliferative effect of a polysaccharide fraction of a 20% methanolic extract of stinging nettle roots upon epithelial cells of the human prostate (LNCaP)." PHARMAZIE, (1999 OCT) 54 (10) 768-71. JOURNAL CODE: 9800766. ISSN: 0031-7144., 1999, XP0008027823

## Description

The present patent of invention has, as main objective, a PHARMACEUTICAL COMPOSITION FOR THE MEDICAL TREATMENT OF BENIGN PROSTATE HYPERPLASIA, ITS METHOD OF PREPARATION and also refers to ITS THERAPEUTIC APPLICATION. From the appearance of this composition, emerges a new and actually unique therapeutic technique, proven effective to pursue the medical management without surgery nor without any type of aggression to the prostate gland, of the BENIGN PROSTATE HYPERPLASIA (enlargement of the prostate).

### GENERALITIES - THE PROBLEM WITHIN THE MEDICAL FIELD

To evaluate the great importance that the problem implies within the medical field and its socio-economic implication world wide, it should be enough to know that benign prostatic hyperplasia (B.P.H) affects approximately 50% of men aged 60. At 80, the estimates are around 80%.

Already in 1988 it was suggested that a man aged 40 with a lifespan of 80 had approximately a 10% chance of having a prostatectomy (total extirpation of prostate) which represents, beyond the anesthetic and surgical risk, not only a castration, but also a future sexual impotence.

Recently, only in the United States, it has been suggested a probability three times greater.

Annually, between 400,000 and 500,000 prostatectomies are performed there for diseases diagnosed as B.P.H, and the total costs (pre-surgery evaluations, hospitalizations, surgical fees, loss of productivity, and treatments for the post-surgical complications) account for a significant percentage of the total monetary cost in health, in the United States.

Throughout the world, including here in Argentina, the same occurs. Finally surgeons as well as urologists begin to accept that the global results of total prostatectomy as well as the trans-urethral (to operate through the urethra) is not as favorable as it was admitted to be till now.

But, on the other hand, doctors also know that so far, despite the fact that no patient wants it, they have no better option than to perform surgery because the new medicaments that appear in the market and the new therapeutic methods of treatment always seem favorable for a short time, later to fail.

### PREVIOUS ART - MEDICAL TREATMENTS

The following are the diverse medical therapies currently being utilized by urologists and surgeons with their patients throughout the world, before surgery is suggested:
*1. Hormonal therapies,* based on the action of androgens on the prostate have turned out to be highly iatrogenic (iatros = physician / genic = induced by). This is because, in addition to finding them not effective in the daily practice, they cause impotence and other undesirable effects.
*2. Finasteride:* (generic name). It inhibits the 5 alpha-reductase. It causes hormonal interference with the patient's testosterone, resulting in loss of erection, libido and even hypertrophy of the mammary gland (gynecomastia). It produces much greater damage than any occasional positive result (for instance diminishing the size of the prostate). Moreover, since it totally lacks residual effects, it must be taken continuously, and its cost is high. In short urologists know that, at most, it is a drug that could be useful for palliative and temporary relief of prostatic symptoms, which they use as a previous solution for an inevitable prostatectomy.
*3. Alpha-adrenergic antagonist receptors:* these medicaments act on the smooth muscle of the bladder neck which continues into the prostatic urethra. In cases of bladder neck hyperplasia they can give good results and this is how we employ them. This muscle is controlled by the Autonomous Nervous System (A.N.S), also known as the Sympathy and Para-sympathic System, which also controls our emotions and sensations. Therefore in the presence of a highly emotive patient who suddenly suffers an anxiety shock due to an over amounting series of worries, his A.N.S can activate sympathic fibers producers of adrenaline and this may finally cause the contraction of the smooth muscle. In such a case the light of the urethra is automatically closed and the urine cannot pass freely; suddenly the patient cannot urinate. In those cases, the alpha-adrenergic antagonist drugs (Terazosine, Tamsulosine, Doxazosine, Alfuzosine) will reestablish the muscle balance allowing the patient to urinate. It must be understood that the positive action of these drugs is performed upon the bladder neck smooth muscle, not having however, effect on the prostatic tumor B.P.H. This one will continue growing and compromising each time the dynamic of urination. We utilize these medicaments when, together with B.P.H. coexist any of those mentioned conditions. These drugs are not harmful, as it is Finasteride, however they may cause tachycardia, dizziness, retrograde ejaculation and mainly, acting on the blood pressure, cause hypotension. That is why, in order to lessen those effects they are taken at bedtime.
*4. Phytotherapeutic agents* (serenoa repens - African pigeons - pollen - etc): are very useful natural drugs not harmful to the organism at all. However acting alone is not quite enough to stop the growing of tumor. We used it toward the end of treatment, as a complementary therapy, only when the Pseudomonas Aeruginosa-Thymus-Prostate compound has attained the main effect.

As part of the Previous Art we mention the application of bacterial polysaccharides on Dermatology, for skin allergic conditions, psoriasis, eczemas, as well as for cheloids, hypertrofic scars and torpidulcus. Also in the anti neoplastic treatments as immunologic buster according to its non specific and desensitizing action.

In that sense, it is known a commercial product named "POLIGRAM", an injectable medication used as anti bacterial with the chosen antibiotic, even as a solvent.

It is understood that in the acute process improves the bactericidal power of antibiotics as reinforces neutrophilia and eosinopenia through the blood.

In renal failure (Nephrology) due to blood transfusion accident it generates dyuresis; in Urology it is employed in papilloma due to virus.

Through its bioplastic action the medicament regenerates the tissues, giving relief to the induration of the cavernous body.

It is a composition containing each 100 ml.:

| | |
|---|---|
| Polysaccharides of Gram Negative Bacteria (Pseudomonas Aeruginosa) | 0,010 g. |
| Total quantification of Carbohydrates expressed as Glucose | 0,076 mg. |
| Sterile physiologic solution s.q.f | 100 ml. |

### SCIENTIFIC FOUNDATION FOR THE USE OF A PHARMACEUTICAL COMPOUND OF PSEUDOMONA AERUGINOSA POLYSACCHARIDES, THYMUS AND PROSTATE FOR THE TREATMENT OF B.P.H.

### Haptenes and Polysaccharides.

Among the incomplete antigens or Haptenes we find the bacterial polysaccharides, and among these is the Pseudomonas Aeruginosa.

Immunology tells us that a Haptene is a tiny non-immunogenic part unable of acting by itself; however when it is added to a protein it forms an antigen. That is why we say it is an incomplete antigen, that is, has not antigen power by itself. And, which is the relationship between a simple polysaccharide and the haptene? The polysaccharide is a haptene, always has been so.

All polysaccharides are or can act as haptenes; however not all the haptenes are polysaccharides.

We hereby are dealing with the Pseudomonas Aeruginosa polysaccharide, which is a haptene and also is an "immune modulator", a substance that acts regulating the activity of the compounds which are dealing with immunity.

In 1955 Heidelberger (1) said that bacteria are true collections of antigens in the most diverse chemical structure; and already in 1936 Landsteiner (2) had discovered that the haptene performed as an incomplete antigen, however, should had been deprived of its protean and lipid fractions - toxic both - it would remain only its polysaccharide fraction which would be useful for therapeutic purposes. At the same time the French Boivin (3) was investigating with lipid-polysaccharides which were causing fever and toxic condition on one side but, on the other hand, they were able to stimulate defenses. Together with other North-American authors he concluded that fever was the responsible for immunologic activity, that is, fever was necessary to get better. Starting from those studies, Puebla in Argentina (4) began his work with the polysaccharide of Pseudomonas Aeruginosa, however, on the contrary he came to the conclusion that the fever was originated by the lipid fraction, which it was necessary to eliminate, because nothing had to do with the improvement of immunity. In 1954 Puebla is the first researcher to obtain the bacterial polysaccharide of Pseudomonas Aeruginosa extract, free of pyrogenic effect, contrary to the hypothesis of those authors mentioned before. He came to the conclusion that the Haptene of P.A. is the polysaccharide of said bacterium who was not acting as a toxic but rather as desensitizer.

The application of this new concept shows the path to the treatment known as Haptenotherapy, opening a new therapeutic hope for Immunology.

Puebla and his followers kept working with the Polysaccharide of P.A., spreading abroad in short time the therapeutic field to other countries, other pathologies and other polysaccharides, (10-12-13-14-17-18-19-20-21-22-23-24 and 25). The investigators managed to demonstrate the outstanding action of P.A., specially so in the diseases involving the connective tissue.

Until that moment, the Argentine physician was convinced that he had found a pure polysaccharide. That was not so. Some years later, the investigator Leyton from Chile (5) concluded a physical-chemical in-depth study of P.A. which confirmed the absence of lipid fractions responsible for the fever and toxic effects; however, at the same time the researcher realized she had found a true compounded polysaccharide with three different elements: a) *the polysaccharide* itself, b) remains of *Nucleic Acids* (AND & ARN) whose action upon tissues improves all metabolic systems explaining the eutrophic effects and well-being of the patient; and c) *Amino-Acids and*/*or Polypeptides.* When its molecular weight is over 5.000 these well known "carriers" may act as antigens, a sort of brick builders of proteins. All those three elements work as *"immune-modulators",* being substances which regulate the whole activity involving Immunity. This fact suggested greater therapeutic benefits. From then on, a broad clinical investigation was on the making; the investigators working with higher doses, so far considered toxic for the patients arrived to the conclusion that bacterial polysaccharides - specially the Pseudomonas Aeruginosa - acting as an immune-modulator participate in various systems:
1. Stimulating the immunity of the sick person. 2. Reestablishing the balance and reactivating the metabolism; and 3, developing bioplastics and fibrolytic effects upon the Connective Tissue.

### 1. Immunity.

Acting as immune-modulators the Pseudomonas Aeruginosa Polysaccharides regulate the activity of the compounds which deal with immunity. These substances stimulate the unspecific defenses blocking the anti-bacteriolysis in the serum of some patients with chronic processes. 6) Waisbren & Brown.

Meier, R. (7) in 1958 tells us about the chemo taxis action of mucus - polysaccharides. That is the attraction they have upon the leukocyte. Meier,R. & Schar,B (8) verify the chemo-taxis activity of gram negative & positive bacterial polysaccharides. Buttler, U. & Thomas, G. in 1956 (9) corroborate that polysaccharides participate in developing the leukocytes migration and also stimulating the phagocytic which is proved because they stimulate neutrophilic, increasing therefore the opsone-cytophagic index. Furthermore, they take part in the transformation of fibroblasts into mobile phagocytes all of which help to reabsorb, in the case of prostate, the necrotic focuses and infarction. Puebla, LC. In 1957 (10) studied the effects of polysaccharides into de resistance to bacterial and viral infections, specially from gram negative bacteria which would be capable to activate the defenses of the human body.

Finally Pillemer, L. (11) discovered the action of bacterial polysaccharides -specially those free of lipids A- upon the Properdine System, and corroborates its bacteriolytic and cytolytic properties as they are capable to block some "immune-globulins" gamma type, (anti-defense fraction).

Between 1955 and 1956, Puebla, CL. Denner, H.O. et al. (12 & 13) working with polysaccharides of Pseudomonas Aeruginosa (P.P.A.) demonstrate its action upon allergic conditions, also its desensitizing effect on the anaphylactic shock and over Arthus & Sanarelli-Schwartzmann Syndrome. Such anti-allergic properties were ascribed by the authors as being due to the desensitizing effect of the Pseudomonas Aeruginosa Hapthene. They explained that this action would activate the defensive specific and unspecific systems, which *are generally uninhibited during the "delayed hyper sensibility processes",* which are ever present in the serum of patients with chronic infections such as chronic prostatitis, viruses, etc.

### 1. Metabolism.

Clinical research tells us that the patient treated with P.P.A. normalizes their metabolic functions.
a) Presents a well being sensation, improves anorexia, mood, asthenia and depression.
b) Normalizes bowel passage and the hydro mechanism (14)
c) Through lipolytic degradation of lipoids it controls their fat in blood. (15 and 16)
d) Enzymatic and co-enzymatic actions in the tricarboxylic cycle of Krebs are improved, therefore helping the glutamic transaminations and favoring new growth of mucus-polysaccharides which are necessary for the mesenchyme and helps the overall detoxicating action in order to normalize the connective tissue role.

### 3. Connective Tissue.

P.P.A. effects on this tissue constitutes the key to understanding its behavior towards BPH or should we say the Benign Prostate Fibroplasia. This action is shown through *(A) bioplastic and (B) fibrinolytic effects.*

Bioplastic effects are due as soon as the fibroblast and the inter-cellular/substance relationship is reestablished and the inflammation and the regeneration of the tissue decreases. It is however also due to the "like cortisone action" of the polysaccharides which inhibits the antigen-antibody reactions. See Leyton (5). Also the authors when studying the bioplastic effects of P.P.A. came to the conclusion that with the improvement of the connective components: fibroblasts, fibrocytes, leio-mio-cytes, collagen fibers and elastine, the regeneration process improved therefore the speed of cure was better and the scar would be cleaner and smoother-not a cheloid.

*The fibrinolytic effects of P:P:A* occurs as the fructose-galactose relationship, which is commonly altered in the process of cheloids and fibrolytic conditions, be reestablished. We must remember that the dangerous infarction and necrotic focuses, grow into the acino producing atrophy.

One of the actions of Pseudomonas Polysaccharides on fibroblasts is to accelerate their transformation into macrophages (mobile phagocytes) which quickly will reabsorb those focuses. Finally in 1966, Derdoy and Puebla (23) investigating fibrosis as result of post-GC infectious and post traumatic urethral strictures, including Peyronie disease, proved the fibrinolytic effects of P.P.A.

These circumstances moved Benaim and Repetto in 1961, (26) to test the action of P.P.A on hyperplastic scars and cheloids which are sequela of burned people. The investigation Publisher in 1979 proved the action of P.P.A in these connective diseases. Results: the study of the dermis showed that, after a three month period of treatment with P.P.A. (IM or IV injections) the "involution" of the fibroblastic process was achieved and a hyaline condition develops upon the thick fibrous tissue; meaning the fibroblastic-hyaline involution as young fibroblasts showed up. This anatomic-pathological condition is clinically coincidental with the cheloid as it began to get softer and thinner. At the same time Benaim sees a deep hyalinization of the collagen fibers. In reference to this important fact we asked ourselves which would be the P.P.A. mechanism of action, and to what result we would arrive using the same procedure in treating the prostate? According to Puebla and Leyton we would imagine that P.P.A. could have an action upon the connective tissue, provoking effects towards its normalization as the Fibroblast-inter collagen substance relationship is restored.

As a consequence, comparing the anatomic-pathological likeness of fibrous scars and mainly the cheloids (being true benign tumors of thick fibrous tissue of the skin, very rich in fibroblasts), with the adeno-fibro-myoma of prostate, I began investigating P.P.A. action on the latter. Authors agree that these two entities are tumors of the connective which involve the fibroblast, and that both are due to a fibroblastic reaction since both have the hyper plastic nodule as the histological sign as well as the fibroblast as the fundamental participating cell in the making of the nodule. In the creation of both tumors, besides the fibrotic tissue participate also collagen fibers, vessels, lymphocytes, etc. Then, the histopathologic disorders are alike, they both invade normal tissue, in prostate and in the skin, as well. Beside, through anatomic-pathological structure both are related to the fibroma or fibro-myoma of the uterus.

### Bibliography

(1)Heidelberger M. "Polysacharides in Biology" Transactions of the 1rst Conference. 27 april, 1955. The Josiah Macy Jr. Foundation.
(2) Landsteiner K. "Specificity of serological reactions", Charles C. Thomas, 1936, Springfield I11., Baltimore, Maryland.
(3) Boivin, A. y Mesrobeaunu L.Revue d'Inmunologie 1: 563, 1935.
(4) Puebla L.C. et al. "Treatment of allergic diseases with the Pseudomona Aeruginosa Polysaccharide" IV Scientific Section, Sociedad Argentina de Pediatria, 9 de agosto de 1955.
(5) Leyton, G. "Bacterial Polysaccharides for therapeutic use" Institute of Bacteriology-.Santiago -Chile - 1966.
(6) Waisbren BA y Brown, I: "A factor in the serum of patients with persisting infection that inhibits the bactericidal activity of normal serum against the organism that is causing the infection" J. of Immunol. 97 (3):431. 1966.
(7) Meir R. "The pharmacological effects of Polysaccharides - Chemistry & Biology of Mucus polysaccharides Ciba Foundation Symposium. J.A. Churchill Ltd. Great Britain, 1958.
(8) Meier, R y Schar,B.; Experientia. 9: 93, 1953. Ref. in(7).
(9) Buttler, U. Y Thomas, G.: Munch. Med. Wschrft, 98:1240, 1956, ref. in (7)
(10) Puebla, L.C. et al. "Acción terapéutica de los polisacáridos de Pseudomona Aeruginosa sobre diversas enfermedades" Dia Médico XXX (48), 1957.
(11) Pillemer, L.; "The nature of Properdine and its interaction with polysaccharide complexes" Ann NY. Academy of Sciences, 66: 233, 1956.
(12)Puebla, L.C. et al. Ref. in (10)
(13) Puebla, L.C. et al. "Tratamiento de dermopatias con un derivado de Pseudomona Aeruginosa" VII Jornadas Argentinas de Pediatria, 1956.
(14) Denner, H.O.; "Acción de los polisacáridos bacterianos sobre el funcionalismo renal". X Jornadas Argentinas de Pediatria, Mar del Plata, Nov. 1959.
(15) Meier R. Y Schuler, cited in (7)1958.
(16) Rivera, R., Comunicación preliminar; Sesión Ordinaria Sociedad Médica de Bio-Bio, Chile, Octubre 2 de 1968.
(17) Denner, H.O., Estudio tecnico presentado al Ministerio de Salud Publica de la prov. de Córdoba (Exp.45.333) y el Ministerio de Asistencia Social y Salud Publica de la Nacion. (Exp. 47.293)
(18) Derdoy, J.B., Garimaldi, J.E.Puebla, L.C., Denner, H.O. y Elena F. De Decca. "Epididimitis Brucelósica. Uso del polisacárido de Brucella Abortus Bang en la intradermoreacción y haptenoterapia". X Congreso Argentino de Urologia, Mendoza, 27-31 de octubre de 1968.
(19) Puebla, L.C., Derdoy,J.B. et al. "Tratamiento de pielonefritis estafilocóccicas con polisacáridos bacterianos". X Congreso Argentino de Urologia, Mendoza, Argentina, 27-31 Octubre de 1968.
(20) Mendieta, L. "Experiencias preliminares con el uso en clinica del polisacárido de Stafilococus Aureus" Sesión Ordinaria Sociedad Médica Bio-Bio, Chile, Oct. 2, 1968.
(21) Garimaldi, J.E., "Pielonefritis y su tratamiento con polisacáridos bacterianos y corticoides" Revista Argentina de Urologia y Nefrologia, 35:88, 1966.
(22) Rivera, R, Suazo, F.L. y Castillo,L., "Comunicación preliminar" Sec. Ordinaria Soc. Médica Bio-Bio, Chile, 1968.
(23) Puebla, L.C y Derdoy, J.B. "Experiencia clínica de la Clinica Regional del Sud". Rio Cuarto, Argentina. 1966-68.
(24) Kabat, E.A. y Mayer, M.M., "Inmunoquímica Experimental". La Prensa Médica Mexicana. México. 1968.
(25) Laje, J.; "Acción terapéutica del polisacárido de Pseudomona Aeruginosa en casos de poliomielitis aguda". "Acción farmacológica de los polisacáridos bacterianos" Clinica Reg. del Sud, Rio Cuarto, Argentina, Nov.1968.
(26) Benaim F. y Repetto, D. "Cicatrices hipertróficas. Actualización y comunicación previa de su tratamiento con el Polisacárido de Pseudomonas Aeruginosa" IV Jornadas de Cirugia Plástica Primer Premio y Medalla de Oro. Rio de La Plata. Diciembre, 1961-1979.

### NOVELTY OF THE INVENTION - MAIN OBJECTIVE

From the previous statement, the main objective of this patent of invention may be ascribed to a Pharmaceutical Compound specially prescribed for the medical treatment of the Benign Prostatic Hyperplasia. A formula which, beside the Gram Negative Germs of the Polysaccharide, (the Pseudomonas Aeruginosa Polysaccharide) also contains two essential elements: Thymus and Prostate (as hydro soluble extract). Clinical results show that a combination of both elements increases the pseudomonas effect and its immune modulator action - bioplastic, fibrinolytic, defensive and eutrophic effects - necessary to successfully act upon the prostatic fibroplasias.

More specifically we are talking of a pharmaceutical compound where, each 1 ml. contains:

| | | |
|---|---|---|
| Polysaccharides of Gram Negative Bacteria | between | 0.05 gr. & 0.002 gr. |
| Thymus (hydro soluble extract) | between | 2 mg. & 0.1 mg |
| Prostate (hydro soluble extract) | between | 1 mg. & 0.01 mg. |
| Total Carbo hydrates (glucose) | between | 2 mg & 0.02 mg. |
| Physiologic solution (bacteria free) s.q.f. | | 1 ml. |

It is understood that the Composition previously indicated uses:
1.- As a Polysaccharide that of Pseudomonas Aeruginosa in the mentioned proportions;
2.- The formula is complemented with the adding of two essential elements in the form of hydro soluble extracts: Thymus & Prostate.

More specifically, in an optimum proportion, it is understood that the invented Composition- which from now on we will call Medicament Compound or simply Cocktail - contains, each 1 ml.

Polysaccharides of Gram Negative Bacteria:

| | |
|---|---|
| Pseudomonas Aeruginosa | 0.005 gr. (= 500 gammas) |
| Thymus (hydro soluble extract) | 0.9 mg. |
| Prostate (hydro soluble extract) | 0.3 mg. |
| Total Carbo hydrates (Glucose) | 0.038 mg. |
| Physiologic Solution (bacteria free) s.q.f. | 1 ml. |

The invention includes the summarized description of the procedures for the preparation of the previously indicated composition. This process is basically composed of three parts:

### PART ONE

### Appropriate method to obtain pseudomona bacteria

The culture of Pseudomonas Aeruginosa will be done in liquid medium in the usual manner, working in rigorous bacterial conditions. For that, we must have a previously typified stock with the corresponding biochemical tests. The culture broth among others, may be a nutritious broth commercially elaborated which can be available in stores specialized in bacterial laboratory products.

It is a must to establish la bacterial growth curve in order to determine the optimum culture time, to obtain later a high concentration of polysaccharides.
From the resulting bacterial mass we must proceed to its washing and later conditioning for its subsequent preservation or extraction.

After they are weighed the bacteria are preserved in freezers till their later use. In all cases the corresponding bacterial controls of the broth are performed through specific biochemical tests, in order to secure they are free of contaminants.

### PART TWO.

### Procedures to obtain bacterial lipoid- polysaccharides.

The bacterial mass must be conditioned before its extraction using techniques with organic solvents. It must be dried and weighed. To weigh the bacteria the culture broth must be ultra centrifuged in order to discard the leftovers. The tare will be obtained weighing the difference between the empty jar (previously weighed) and the jar with bacterial sediment.

A phenol solution in optimum concentration must be prepared in order to obtain the maximum efficiency and then, we must use the liquid phase discarding the bacterial remnants.

### PART THREE.

### Procedures to separate the lipoid fraction in order to obtain the pseudomona aeruginosa in its state of maximum purity.

We proceed to the separation of the lipoid fraction to obtain the pure polysaccharide (desensitizer haptene).

To such effects we utilize hydrolysis to eliminate the toxic fraction (lipoid) and then it must be ultra centrifuged to finally obtain the polysaccharide in its state of highest purity (lyophilize).

In addition, the present patent of invention also refers to the medical treatment of the Benign Prostatic Hyperplasia. This patent consists in the Therapeutic Application of this Medical Composition.

### SAMPLES OF APPLICATION

### CLINICAL INVESTIGATION CARRIED OUT WITH 200 PROSTATIC PATIENTS BETWEEN THE YEARS 1997 AND 2002 IN THE MEDICAL CENTER OF THE BARREIRO FOUNDATION IN BUENOS AIRES.

Upon my return from Liberia, Africa Occidental in 1983, after having work as physician in the jungle,

I set residence in Buenos Aires. Being myself a medical practitioner fully familiarized with the clinical observation of the daily practice, where the teacher is the balance of failure and accomplishment, the laboratory was my consulting office and the materials my patients.

Fully aware that P.P.A. do not have iatrogenic effects, neither Thymus or Prostate as hydro soluble extracts, even in the most older patients, in 1984 I began my experience with the use of this medicament. The first thing was to find out, (a): the proportion of each element in the formula, b): then the adequate initial dosage and, (c): the way of dispensing the medicament.

From there on several thousands of prostate patients that were ready for surgery came into our Foundation to receive medical treatment; each one would convey us new knowledge. The results were more and more revealing, until in the year 1996 I started to build up a protocol and the following year, 1997 I began the investigation. This would allow us to evaluate, inform and share results with my colleagues.

### THE INVESTIGATION

### Group A

As they entered the Center, out of 640 patients with urinary obstruction symptoms admitted from January 2, 1997 to August 31, the same year, a group of 200 aged between 56 and 92, and prostate volume between 45 and 158 cc. were selected at random after they passed laboratory tests. Exclusion criteria: prostate cancer, urinary stasis leading to renal failure, detrusor instability or decompensation, cerebro vascular illness, severe neurological or psychiatric disorders, urethral strictures, large bladder diverticulums, bacterial prostatitis, neurological bladder and previous prostate surgery, were all excluded.

On January 2, 1997 the real investigation started with the first group of 11 patients. Further laboratory and diagnosis tests were performed during a week. This group commenced the randomized, double blind, placebo controlled treatment on January 10 (we shall call it Day Zero). The admission of patients for this trial closed on August 31 of the same year. Each patient was treated during 120 days. On December 31 the last patient was discharged. That same day the test ended.

Baseline control: complete physical exam; digital rectal exam of prostate, specifically its size and grade of hardness; clinical history including any acute urinary retention, family background past and present illnesses, routine prostatic secretion test, blood and urine with sediment, and fecal exam; trans-rectal ultrasound; fluxometry (maximum flow) and residual urine obtained by catheter.

In order to correct any anomaly, distant sepsis focuses (sinus, tonsils and teeth) were checked out as routine, as well as the alimentary, bowel movements and urinary habits.

The 200 patients were divided into 2 groups of 100 patients each: the blue Placebo group-(BPG) whose medicament was identified with a blue label and the green group (GG), treated with P.A.P. was identified with a green label.

All the patients were explained the objectives and importance of the study. They were also informed that half of them would receive the complete treatment from the first day. The other half would be injected with a sterile solution but only during the first 45 days; during which period they would not suffer any risk or inconvenience. They were also told that starting day 46 they would receive their real treatment (with P.A.P.) and as an incentive prize for their collaboration, the total cost of their treatment (Placebo group) including medicaments would be paid by the Foundation. Finally they were told that neither the doctors nor any patient would know until day 45 who was receiving the placebo or the P.A.P.

All the 200 patients approved and an informed consent was signed.

Accordingly, the blue Placebo group received 1 ml. subcutaneous (SC) injections of normal saline solution from day zero up to day 45. That same day the randomization code was broken and those patients were told that, starting the following day 46 (in fact day zero for the trial) they had been included in the green P.A.P. group, chosen to receive their real treatment with our cocktail, free of charge.

The reason why there were only 45 days with Placebo was that the positive effects of the cocktail appear approximately within a period of 20 days from day zero. That is why, since we allowed a little more than twice of such a time, it was considered enough to mark the difference and get a convincing result.

### THE TREATMENT:

The treatment began on day zero for 100 patients of the green PAP group. Treatment for 100 patients of the blue Placebo group started on day 46 (day zero for them), a total of 200 participants.

From there on, these two groups received the Pseudomonas Aeruginosa-thymus-prostate cocktail, the following manner:

2 ml. SC injections per day (equivalent to 1000 gammas of Pseudomonas Aeruginosa Polysaccharide) at any time during 20 continuous days, subcutaneous SC injections in the upper arms and legs, always rotating, using 1 cc. disposable insulin Terumo syringes and needles.

From day 21 till day 45 it was administered 1 ml. of P.P.A. (500 gammas), 6 days per week, Monday *thru Saturday. Sunday to rest.*

Then, starting day 46 until day 66, 1 ml. was applied (500 gammas), reducing to 5 injections a week, from Monday to Friday. Saturday and Sunday to rest. From day 67 to 90 we continue with 1 ml. (500 gammas) only 4 days a week: Monday, Wednesday, Friday and Sunday.

Finally, from day 91 to day 120, likewise (500 gammas) daily, only Monday, Wednesday and Friday.

That same day, after four months, the treatment ended and the patients were discharged.

### While the test lasted, the injections were not suspended for any reason.

### THE RESULTS:

The injections of the medicament are painless and without any undesirable effect, as thousand of treated patients assure.

Because of being more comfortable we had chosen SC injections, however I.M. could be also administered, even the I.V. way with identical results.

They were never reported allergic reactions, fever or any other undesirable side-effect.

The whole treatment was performed under ambulatory basis. Did not interfere with any medicament the patient might be taken simultaneously (anti diabetic, anti hypertensive, Central nervous system medication, Autonomic nervous system, Cardio-respiratory drugs or else. Likewise, did not interfere with appetite, food, sleep, weigh and the patient hormonal system.

In short, the medicament is well tolerated regarding side effects, showing no difference with those patients who received placebo.

As it is routine, all patients were initiated on intensive bladder exercises in order to restore Detrusor strength.

### PATIENTS WHO ABANDONED THE TRIAL.

Out of the 100 patients of the blue placebo group, 12 suspended the trial before day 45. From these, 6 for family reasons decided to go on surgery (days 9,12,19,27, 29 & 33, from day zero); 2 were sent to a psychiatrist with depression, (days 23 & 30); 1 had haematuria due to urinary lithiasis (day 8); 1 broke his hip (day 15) and 2 did not return ever (days 19 % 26).

Out of the 100 patients of the green PAP group, 17 suspended treatment: 2 because of car accident(days 55 & 69), however both returned during the year 1999 to complete the treatment; 4 for economic reasons (days 36, 46, 65 & 79); 2 could not follow the prescribed diet due to social reasons and went to surgery; (days 21 & ?); 2 patients were in a hurry to get results and withdraw (days 16 & ?); another 2 because they felt well enough to consider themselves cured (days 80 & 93); 2 businessmen left the trial as they felt well and went overseas (days 53 & 100); 1 had lung cancer (day 109) and finally 2 left the treatment without explanation (days 16 & 49).

All the 200 patients of both groups were three time evaluated: on day zero before the study began; on days 45 and 120 (as they ended the treatment). The original Boyarsky's IPSS (International Prostate Score System) International Committee for B.P.H., June 1993, was used in this study.

We provide the following results:

| **SAMPLE OF APPLICATIONS** | | | | **TABLES OF RESULTS** | | | | |
|---|---|---|---|---|---|---|---|---|
| Chart 1. Mean values of symptoms in the groups treated with the cocktail P.P.A.-Thymus-Prostate, and with Placebo, evaluated according to prostate score symptoms. | | | | | | | | |
| | | | | | | | | |

| | (Green PAP group) n = 100 patients treated with cocktail | | | (Blue P. group) n = 100 patients treated with Placebo | | (ex Placebo group) (*2) n = 100 treated with cocktail | | |
|---|---|---|---|---|---|---|---|---|
| | | | | | | | | |

| | Day Zero | Day 45 | Day 120 | Day Zero | Day 45 (*1) | Day Zero | Day 45(*3) | Day 120(*4) |
|---|---|---|---|---|---|---|---|---|
| Nocturia | 2.1 | 1.1 | 0.6 | 2.2 | 2 | 2 | 1.2 | 0.7 |
| Daytime frequency | 1.7 | 1.1 | 0.8 | 1.6 | 1.4 | 1.4 | 1 | 0.5 |
| Hesitancy | 1.8 | 1.2 | 0.5 | 1.9 | 1.7 | 1.7 | 1.4 | 0.8 |
| Intermittence | 1.3 | 0.7 | 0.4 | 1.5 | 1.4 | 1.4 | 0.8 | 0.5 |
| Terminal dribbling | 1.2 | 0.8 | 0.3 | 1.2 | 1.1 | 1.1 | 0.9 | 0.4 |
| Urgency | 1.7 | 1.1 | 0.4 | 1.7 | 1.8 | 1.8 | 1.4 | 0.8 |
| Weak Stream | 1.3 | 0.9 | 0.4 | 1.2 | 1.1 | 1.1 | 0.6 | 0.3 |
| Dysuria | 0.9 | 0.5 | 0.4 | 0.8 | 0.8 | 0.8 | 0.6 | 0.4 |
| | | | | | | | | |
| Sensation of incomplete voiding | 1.4 | 0.8 | 0.4 | 1.4 | 1.7 | 1.7 | 0.7 | 0.5 |
| Total points | 13.4 | 8.2 | 4.2 | 13.5 | 13.0 | 13.0 | | 4.9 |
| Q max | 8.5 | 14.4 | 18.6 | 7 | 7.2 | 7.2 | 15.1 | 18.9 |
| Residual urine Volume (ml.) | 155 cc | 92 cc. | 35 cc. | 141 cc | 148 cc | 148 cc. | 87 cc | 45 cc. |
| Prostatic specific Antigen (PSA) | 6.1 | 4.2 | 2.5 | 6.4 | 6.3 | 6.3 | 5.1 | 2.5 |
| Prostatic Volume cc 60 gr. | 60 gr. | ----- | 49 gr. | 62 gr. | 63 gr. | 63 gr. | ----- | 50 gr. |
| | | | | | | | | |

| | | | | | | | | |
|---|---|---|---|---|---|---|---|---|
| (*1). This day 45° ended the trial with Placebo. | | | | | | | | |
| (*2).This group received Placebo during 45 days. Now, from day 0 (day 46° since the study started) they were treated with P.P.A cocktail. | | | | | | | | |
| Its first evaluation was on day 45° (*3), and the 2^{nd}. evaluation it was on day 120°.(*4) | | | | | | | | |

Are well known the ups and downs of B.P.H., including the fluctuation of symptoms with self-generated deterioration and spontaneous recoveries, periods of stability followed for more deterioration and so on. All of which makes, in our point of view necessary to evaluate the importance of the complementary therapies for the medical management of B.P.H.

On the other hand, we consider the residual urine as a sign of abnormal function of the bladder, more than as a direct result of urinary obstruction. That is why we teach our prostatic patients not only how to eat properly, but also to perform bladder exercises using such muscles as the Detrusor, elevator of anus, the perineal and abdominal muscles; all of which will quickly improve the problems regarding to the contraction strength of their bladder and, as a consequence, also to its residual urine as well as its grade of intermittence, hesitation and maximum flow (strength of its urine stream)

### Follow - up

Knowing that BPH is a chronic and potentially progressive disease, it is important that the results of any medical treatment be investigated and evaluated in a long period of time.

To fulfill this principle, all the patients who participated in the trial were requested to return to the Foundation into 12, 24, 36, 48, and 59 months starting from the day they ended the treatment, in order to be checked out and evaluated. It was a five years follow-up period.(day 120° - chart 2)

**Chart 2 - of the follow-up.**

| **Returning year** | **1998** | **1999** | **2000** | **2001** | **2002** |
|---|---|---|---|---|---|
| Months elapsed since Day 120 | 12 | 24 | 36 | 48 | 59 |
| Number of Returning patients (*) | 198 | 164 | 138 | 124 | 58 |
| Nocturia | 0,6 | 0,8 | 0,7 | 1,01 | 1,00 |
| Daily frequency | 0,7 | 0,6 | 1,00 | 1,02 | 0,95 |
| Hesitancy | 0,6 | 0,9 | 1,20 | 1,17 | 1,50 |
| Intermittence | 0,5 | 0,4 | 0,8 | 0,6 | 0,6 |
| Terminal dribbling | 0,3 | 0,6 | 0,4 | 0,3 | 0,5 |
| | | | | | |

| **Returning year** | **1998** | **1999** | **2000** | **2001** | **2002** |
|---|---|---|---|---|---|
| Urgency | 0,9 | 1,00 | 0,8 | 0,8 | 1,2 |
| Weak stream | 0,6 | 0,8 | 0,9 | 0,8 | 0,7 |
| Dysuria | 0,5 | 0,5 | 0,7 | 0,4 | 0,8 |
| Sensation of incomplete voiding | 0,4 | 0,4 | 0,5 | 0,6 | 0,5 |
| Q max (ml/sec) | 16,30 | 14,92 | 16,90 | 15,60 | 15,39 |
| Residual orine Volume (ml) | 46 cc | 48 cc | 47 cc | 49 cc | 45 cc |
| Prostate specific Antigen (P.S.A.) | 1,50 | 2,30 | 2,15 | 0,90 | 2,60 |
| Prostate volume (cc) | 52 gr. | 53 gr. | 49 gr. | 54 gr. | 50.5 gr. |

| | | | | | |
|---|---|---|---|---|---|
| (*) Although not all patients returned exactly on time a year later, many of them did it some time later at any moment, during the five following years. They said they were feeling well. The percentage falling in this category is hard to obtain. | | | | | |

In spite that during the present study was found an evident improvement in the irritating and obstructive symptoms, could not be found significant differences regarding the volume of the prostate between the placebo group and those patients treated with the recommended medication. In fact this may result in a contradiction with the overall improvement.

However, analysis of large bases of clinical information provide us the evidence that prostate volume is not consequent neither with the strictness of the urethral obstruction nor with symptoms of Prostatism. See: (27) Lytton B, Emery JM, Harvard BM: "The incidence of benign prostatic obstruction" J.Urol 99: 639-645, 1968 (28) Turner Warwick T et al: "A uro-dynamic view of prostatic obstruction and the results of prostatectomy" B J.

Urol 45: 631-645, 1973. (29) Chapman I, Lapi N, Fethiere W: "Prostatic enlargement and low urinary tract obstruction". Geriatrics 19: 231-234, 1964.

In other way, Trans-rectal ultrasound give us the volume of the gland, however nothing is said about what is going on inside which seems to be essential, because it is not the volume but the urinary obstruction what it really counts.

The road is open to future investigations in order to add to some unsolved issues. Such an investigation would be important because would bring more light regarding the therapeutic potential of this cocktail. Also the possible reducing effects over the uterine fibroma and its periodic hemorrhages, which would be correlated to the same mechanism of action; furthermore, would confirm or not its positive action on B.P.H., the main issue of this study.

Notwithstanding the obvious difficulties, conclusion of the follow up tell us that the treatment with the cocktail of P.P.A.-Thymus-Prostate have a residual power, that is, its positive effects continues after the treatment is ended, as long as the patient carry on with the hygienic-dietetic habits and with the bladder exercises prescribed in due time.

In short: after his four months time of treatment the period of well being for the prostatic patient may last up to several years without a need for a reinforcement dose. This is according to our archives. As a routine, the Center dates for an interview each year to the patient but when he fails due to the fact he feels well and he lost contact with us during three or four years, he may find his urination deteriorated. In such a case, generally a reinforcement dose of 1cc. S.C daily, during 20 to 40 following days will return the health to the patient.

Notwithstanding, we can see patients after 10 or more years without contacting the Center living in good urinary condition.

### CONCLUSSIONS

The mentioned therapeutic formula shows clinical as well as statistically a significant improvement in the symptoms and signs of B.P.H. within the three or four weeks after treatment, achieving its top effect in quite less of 4 months. The prima facie results of the present study lead us to think that the fibrolytic action of the medicament works on the prostatic stroma slowing its invasion over the parenquima thus reestablishing the fibroblast-inter collagen substance relationship. This fundamental effect has been demonstrated through the long investigations of Puebla, Rivera, Derdoy and other.(see 4.5.9.12.13.15.16.17.18.19.20.21.& 22)

A rise in the elements of the prostatic stroma has been described as the most prominent morphological difference between B.P.H. and the normal prostatic tissue. S (30) Mac Neal JE:
"Origin and evolution of benign prostatic enlargement" Invest Urol 15:340-345, 1978.

This process would give us the clinical explanation of why as a new rectal examination is performed approximately 5 weeks after the treatment with our cocktail, we palpate a gland with softer points (jelly like), being this softness felt on the surface, however also felt in depth with the tip of our index finger. This is a softness which is corresponding with the formerly described fibro-hyaline involution, which in time seems to spread all over the gland. Then, logic tells us that the fibrotic nodule, a participating element of the fibro-leio-adeno-myoma starts to get soft
- as occurs with the cheloid. Finally, in this case the pressure upon the urethra begins to decrease, and the symptoms and signs of the disease automatically improve. We refer to the studies and findings of Benaim (see 26).

**ADVANTAGES OVER OTHER MEDICAMENTS USED AT PRESENT FOR THE TREATMENT OF B.P.H.**

| | | | |
|---|---|---|---|
| | **Treated with a compound P.P.A.-Thymus-Prostate** | **Treated with Finasteride (1)** | **Treated with Placebo** |
| **Differences** | ***Up to 17 weeks of treatment*** | ***Up to 24 weeks of treatment*** | ***Up to 45 days of treatment*** |
| Nocturia | 0,6 | 1,5 | 2 |
| Daytime Frequency | 0,8 | 1,4 | 1,4 |
| Hesitancy | 0,5 | 0,5 | 1,7 |
| Intermittence | 0,4 | 0,7 | 1,4 |
| Terminal dribbling | 0,3 | 0,4 | 1,1 |
| Urgency | 0,4 | 0,7 | 1,8 |
| Weak stream | 0,4 | 0,6 | 2 |
| Dysuria | 0,4 | 0,4 | 0,8 |
| Sensation of incomplete voiding | 0,4 | 0,9 | 1,7 |
| | | | |
| Total points (I-PSS) | 4,2 | 7,1 | 13,9 |
| Q max. (ml./sec) | 18,6 | 10,3 | 7,2 |
| Residual Urine volume (m.l.) | 35 cc | 76 cc | 148 cc |
| Prostatic Specific Antigen (ng.%) | 2,5 | 2,9 | 6,3 |
| Prostatic volume (grs.) | 49 gr. | 35 gr. | 63 gr. |
| | | | |
| | | | |
| Cost of medicament | cheaper | Much more expensive | ----------- |
| Duration of treatment | 120 days | Must be continuous | ----------- |
| Residual effect | Up to 5 years | none | ----------- |
| Undesirable secondary effects | none | Diminished libido Diminished sexual function Possible mammary hyperplasia | ----------- |

| | | | |
|---|---|---|---|
| (1) "Urodynamic effects of Finasteride in the treatment of Bladder outlet obstruction due to Benign Prostatic Hyperplasia" Teuvo L Tammela and Matti J.Kontturi. Journal of Urology-Vol. 149, 342-344. February 1993. | | | |

It has been described and exemplified the nature and the main objective of the present invention, and also the advantages upon other medicaments used at present for the same disease; as well as the way in which the invention can be put into practice, it is hereby claimed the ownership and the exclusive legal rights:

## Claims

1. **A PHARMACEUTICAL COMPOUND FOR THE MEDICAL TREATMENT OF THE BENIGN PROSTATE HYPERPLASIA,** duly **characterized** because it is a Pharmaceutical Composition in which each 1 ml. contains:
- Polysaccharides of Gram Negative Bacteria between 0.05 gr. & 0.002 gr.
- Thymus (hydro soluble extract) between 2 mg. & 0.1 mg
- Prostate (hydro soluble extract) between 1 mg. & 0.01 mg.
- Total Carbo hydrates (glucose) between 2 mg & 0.02 mg.
- Physiologic solution (bacteria free) s.q.f. 1 ml.

2. **A PHARMACEUTICAL COMPOUND FOR THE MEDICAL TREATMENT OF THE BENIGN PROSTATE HYPERPLASIA,** according to claimed in 1, duly **characterized** because the Polysaccharide is a Polysaccharide of Pseudomonas Aeruginosa in the indicated proportions.

3. **A PHARMACEUTICAL COMPOUND FOR THE MEDICAL TREATMENT OF THE BENIGN PROSTATE HYPERPLASIA,** according to claimed in 1, duly **characterized** because the Compound contains each 1 ml:
- Polysaccharides of Pseudomonas Aeruginosa 0.005 gr.
- Thymus (hydro soluble extract) 0.9 mg.
- Prostate (hydro soluble extract) 0.3 mg.
- Total Carbo hydrates (Glucose) 0.038 mg.
- Physiologic Solution (bacteria free) s.q.f. 1 ml.

4. **THE PREPARATION METHOD** of the composition claimed in 1, duly **characterized** because it includes an appropriate method to culture Pseudomonas bacteria in the following conditions:
a) the culture of the mentioned polysaccharide is performed in a liquid medium in the same specialized manner as it is usually done, taking always into consideration that work must be carried out under the most rigorous conditions of bacteriological asepsis.
b) It is necessary to obtain an appropriate stock, previously typified with the corresponding biochemical tests.

5. **PREPARATION METHOD,** according to claimed in 4, duly **characterized** because the culture stock to use, will be chosen among the nutritious cultured broths which are available at laboratories producing commercial bacteriological products.

6. **PREPARATION METHOD,** according to claimed in 4, duly **characterized** because it is essential to establish the Bacteria Growing Curves in order to know the optimum culture time to obtain thereafter a high concentration of polysaccharide.

7. **PREPARATION METHOD,** according to claimed in 4, duly **characterized** because the resulting bacterial mass will be carefully washed and conditioned. After been weighed, bacteria will be stored in freezer until they are needed.

8. **PREPARATION METHOD,** according to claimed in 4, duly **characterized** because in all cases the corresponding bacteriological control of the stock will be performed through specific biochemical tests in order to ensure they are bacteria free.

9. **PREPARATION METHOD,** according to claimed in 4, duly **characterized** because the bacterial mass must be conditioned first to then carry on to its extraction, using techniques with organic solvents. Thereafter the mass is duly dried and weighed. To weigh the bacteria the culture broth must be ultra-air-centrifuged in order to discard the leftovers.

10. **PREPARATION METHOD,** according to claimed in 4, duly **characterized** because we proceed to weigh the tare which is obtained weighing the difference between the empty jar (previously weighed) and the jar with bacterial sediment. Thereafter, a phenol solution is prepared in optimum concentration in order to obtain the maximum efficiency and then, we must use the liquid phase but discarding the bacterial remnants. Finally, we proceed to the separation of the lipoid fraction to obtain the pure polysaccharide (desensitizer haptene).To such effects we utilize hydrolysis to eliminate the toxic fraction (lipoid) and then it must be ultra centrifuged to finally obtain the polysaccharide in its state of highest purity. To lyophilize.

11. **USE OF THE PHARMACEUTICAL COMPOUND ACCORDING TO CLAIM 1 FOR THE MANUFACTURE OF A MEDICAMENTE FOR THE TREATMENT OF BENIGN PROSTATIC HYPERPLASIA,** duly **characterized** because it consists in the use of a Pharmaceutical Compound in which each 1 ml. contains:
- Polysaccharides of Gram Negative Bacteria between 0.05 gr. & 0.002 gr
- Thymus (hydro soluble extract) between 2 mg. & 0.1 mg
- Prostate (hydro soluble extract) between 1 mg. & 0.01 mg.
- Total Carbo hydrates (glucose) between 2 mg & 0.02 mg.
- Bacteria free physiologic solution s.q.f. 1 ml.

12. **USE OF THE PHARMACEUTICAL COMPOUND ACCORDING TO CLAIM 1 FOR THE MANUFACTURE OF A MEDICAMENTE FOR THE TREATMENT OF BENIGN PROSTATIC HYPERPLASIA** according to claimed in 11, duly **characterized** because the Composition contains each 100 ml.:
- Polysaccharide of Pseudomonas Aeruginosa 0.005 gr.
- Thymus (hydro soluble extract) 0.9 mg.
- Prostate (hydro soluble extract) 0.3 mg.
- Total Carbo hydrates (Glucose) 0.038 mg.

## Patentansprüche

1. **EINE PHARMAZEUTISCHE VERBINDUNG FÜR DIE MEDIZINISCHE BEHANDLUNG DER BENIGNEN PROSTATAHYPERPLASIE, dadurch gekennzeichnet, dass** sie eine Pharmazeutische Zusammensetzung ist, in welcher pro 1 ml folgendes enthalten ist:
- Polysaccharide aus gramnegativen Bakterien zwischen 0.05 gr. und 0.002 gr.
- Thymus (wasserlöslicher Extrakt) zwischen 2 mg. und 0.1 mg
- Prostata (wasserlöslicher Extrakt) zwischen 1 mg. und 0.01 mg.
- Gesamt-Kohlenhydrate (Glukose) zwischen 2 mg und 0.02 mg.
- Physiologische Lösung (bakterienfrei) s.q.f. 1 ml.

2. **EINE PHARMAZEUTISCHE VERBINDUNG FÜR DIE MEDIZINISCHE BEHANDLUNG DER BENIGNEN PROSTATAHYPERPLASIE** nach Anspruch 1, **dadurch gekennzeichnet, dass** der Polysaccharid ein Polysaccharid aus Pseudomona aeruginosa in den genannten Proportionen ist.

3. **EINE PHARMAZEUTISCHE VERBINDUNG FÜR DIE MEDIZINISCHE BEHANDLUNG DER BENIGNEN PROSTATAHYPERPLASIE** nach Anspruch 1, **dadurch gekennzeichnet, dass** die Verbindung pro 1 ml folgendes enthält:
- Polysaccharide aus Pseudomona aeruginosa 0.005 gr.
- Thymus (wasserlöslicher Extrakt) 0.9 mg. - Prostata (wasserlöslicher Extrakt) 0.3 mg.
- Gesamte Kohlenhydrate (Glukose) 0.038 mg.
- Physiologische Lösung (bakterienfrei) s.q.f. 1 ml.

4. **DIE ZUBEREITUNGSMETHODE** der Zusammensetzung nach Anspruch 1, **dadurch gekennzeichnet, dass** es ein geeignetes Verfahren zur Bildung von Pseudomona-Bakterien-Kulturen unter folgenden Bedingungen umfasst:
a) die Kultur des erwähnten Polysaccharids wird in einem flüssigen Mittel in derselben spezialisierten Weise wie üblich ausgeführt, immer unter Berücksichtigung, dass diese Arbeit unter den striktesten Bedingungen bakteriologischer Asepsis durchgeführt werden muss.
b) Es ist notwendig, ein entsprechendes Lager zu erhalten, das mit den entsprechenden biochemischen Tests vorverkörpert wurde.

5. **ZUBEREITUNGSMETHODE** nach Anspruch 4, **dadurch gekennzeichnet, dass** der zu verwendende Kulturbestand unter den nahrhaften kultivierten Brühen ausgewählt wird, die bei den Laboratorien, die kommerzielle bakteriologische Produkte herstellen, verfügbar sind.

6. **ZUBEREITUNGSMETHODE** nach Anspruch 4, **dadurch gekennzeichnet, dass** es wesentlich ist, die Bakterienwachstumskurven zu erstellen, um die optimale Kulturperiode zu kennen und danach eine hohe Polysaccharidenkonzentration zu erhalten.

7. **ZUBEREITUNGSMETHODE** nach Anspruch 4, **dadurch gekennzeichnet, dass** die entstehende Bakterienmasse sorgfältig gewaschen und konditioniert wird. Nach Abwiegen werden die Bakterien im Gefrierfach gelagert bis sie benötigt werden.

8. **ZUBEREITUNGMETHODE** nach Anspruch 4, **dadurch gekennzeichnet, dass** in allen Fällen die entsprechende bakteriologische Kontrolle des Lagers über spezifische biochemische Tests durchgeführt wird, um sicherzustellen, dass sie bakterienfrei sind.

9. **ZUBEREITUNGSMETHODE** nach Anspruch 4, **dadurch gekennzeichnet, dass** die Bakterienmasse vor ihrer Entnahme zuerst konditioniert werden muss, unter Benutzung von Techniken mit organischen Lösungsmitteln. Danach wird die Masse entsprechend getrocknet und gewogen. Zum Wiegen der Bakterien muss die Kulturbrühe ultra-luftzentrifugiert sein, um Reste auszustossen.

10. **ZUBEREITUNGSMETHODE** nach Anspruch 4, **dadurch gekennzeichnet, dass** wir die Tara wiegen, was so erreicht wird, indem die Differenz zwischen dem leeren Gefäss (vorgewogen) und dem Gefäss mit dem Bakteriensediment gewogen wird . Danach wird eine Phenollösung in optimaler Konzentration vorbereitet, um den Höchstwirkungsgrad zu erhalten und dann müssen wir die flüssige Phase verwenden, jedoch indem die Bakterienüberreste ausgesondert werden. Schließlich gehen wir zur Trennung der Lipoidfraktion über, um den reinen Polysaccharid (Haptene-Desensibilisator) zu erhalten. Hierzu verwenden wir eine Hydrolyse, um die toxische Fraktion (Lipoid) zu beseitigen und dann muss es ultra-zentrifugiert werden, um schließlich den Polysaccharid in seinem höchstreinen Zustand zu erhalten. Zum Gefriertrocknen.

11. **VERWENDUNG DER PHARMAZEUTISCHEN VERBINDUNG NACH ANSPRUCH 1 ZUR HERSTELLUNG VON EINEM MEDIKAMENT FÜR DIE BEHANDLUNG DER BENIGNEN PROSTATAHYPERPLASIE, dadurch gekennzeichnet, dass** es aus der Verwendung einer Pharmazeutischen Verbindung besteht, bei welcher pro 1 ml folgendes enthalten ist:
- Polysaccharide aus gramnegativen Bakterien zwischen 0.05 gr. und 0.002 gr
- Thymus (wasserlöslicher Extrakt) zwischen 2 mg. und 0.1 mg
- Prostata (wasserlöslicher Extrakt) zwischen 1 mg. und 0.01 mg.
- Gesamte Kohlenhydrate (Glukose) zwischen 2 mg und 0.02 mg.
- Bakterienfreie Physiologische Lösung s.q.f. 1 ml.

12. **VERWENDUNG DER PHARMAZEUTISCHEN VERBINDUNG NACH ANSPRUCH 1 ZUR HERSTELLUNG VON EINEM MEDIKAMENT FÜR DIE BEHANDLUNG DER BENIGNEN PROSTATAHYPERPLASIE NACH ANSPRUCH 11, dadurch gekennzeichnet, dass** die Zusammensetzung pro 100 ml folgendes enthält:
- Polysaccharide aus Pseudomona aeruginosa 0.005 gr.
- Thymus (wasserlöslicher Extrakt) 0.9 mg.
- Prostata (wasserlöslicher Extrakt) 0.3 mg.
- Gesamt-Kohlenhydrate (Glukose) 0.038 mg.

## Revendications

1. Composé pharmaceutique pour le traitement médical de l'hyperplasie bénigne de la prostate, dûment **caractérisé en ce qu'**il consiste en une composition pharmaceutique où chaque 1 ml. contient:
- Polysaccharides de Bactéries Gram négatives entre 0.05 gr & 0.002 gr.
- Thymus (extrait hydrosoluble) entre 2 mg & 0.1 mg.
- Prostate (extrait hydrosoluble) entre 1 mg & 0.01 mg.
- Total de Carbohydrates (glucose) entre 2 mg & 0.02 mg.
- Solution physiologique (dépourvue de bactéries) s.q.f. 1 ml.

2. Composé pharmaceutique pour le traitement médical de l'hyperplasie bénigne de la prostate, conformément à la revendication 1, dûment **caractérisé par le fait que** le Polysaccharide est un Polysaccharide de Pseudomonas Aeruginosa dans les proportions indiquées.

3. Composé pharmaceutique pour le traitement médical de l'hyperplasie bénigne de la prostate, conformément à la revendication 1, dûment **caractérisé en ce que** le composé contient pour chaque 1 ml:
- Polysaccharides de Pseudomonas Aeruginosa 0.005 gr.
- Thymus (extrait hydrosoluble) 0.9 mg.
- Prostate (extrait hydrosoluble) 0.3 mg.
- Total de Carbohydrates (Glucose) 0.038 mg.
- Solution physiologique (sans bactéries) s.q.f.1 ml.

4. Méthode de préparation de la composition de la revendication 1, dûment **caractérisée en ce qu'elle** inclut une méthode appropriée pour la culture des bactéries Pseudomonas dans les conditions suivantes:
a) la culture du polysaccharide mentionné est réalisée dans un milieu liquide de la même manière spécialisée que celle qui s'effectue habituellement, tout en tenant en compte le travail devant s'effectuer dans les conditions d'asepsie bactériologique les plus rigoureuses possibles.
b) II est nécessaire d'obtenir une réserve appropriée, préalablement typifiée par les tests biochimiques correspondants.

5. Méthode de préparation, conformément à la revendication 4, dûment **caractérisée en ce que** la réserve de culture à utiliser, va être choisie parmi les bouillons nutritifs cultivés qui sont prévus pour les laboratoires de production des produits bactériologiques commerciaux.

6. Méthode de préparation, conformément à la revendication 4, dûment **caractérisée en ce qu'**elle est essentielle pour établir les Courbes de Bactéries Croissantes afin de connaître le temps de culture optimal pour obtenir ensuite une concentration élevée de polysaccharides.

7. Méthode de préparation, conformément à la revendication 4, dûment **caractérisée par le fait que** la masse bactérienne résultante sera soigneusement lavée et conditionnée. Une fois pesées, les bactéries seront stockées dans un congélateur jusqu'à leur utilisation.

8. Méthode de préparation, conformément à la revendication 4, dûment **caractérisée en ce que**, dans n'importe quel cas, le contrôle bactériologique correspondant de la réserve se réalisera au moyen de tests biochimiques spécifiques de manière à assurer qu'elle est dépourvue de bactéries.

9. Méthode de préparation, conformément à la revendication 4, dûment **caractérisée en ce que** la masse bactérienne doit être, en premier lieu conditionnée, puis on procèdera à son extraction en utilisant des techniques avec des solvants organiques. La masse est ensuite dûment séchée et pesée. Pour peser les bactéries, le bouillon de culture doit être ultra centrifugé par air afin d'éliminer les résidus.

10. Méthode de préparation, conformément à la revendication 4, dûment **caractérisée en ce que** l'on procède à peser la tare qui est obtenue en pesant la différence entre le récipient vide (pesé antérieurement) et le récipient contenant le sédiment bactérien. À continuation, une solution phénol est préparée avec une concentration optimale afin d'obtenir l'efficacité maximale puis la phase liquide doit être utilisée mais en éliminant les restes bactériens. Finalement, on procède à la séparation de la fraction lipoïdique afin d'obtenir le polysaccharide pur (haptène désensibilisateur). Pour obtenir de tels effets, on utilise une hydrolyse pour éliminer la fraction toxique (lipoïdique) et celle-ci doit être postérieurement ultracentrifugée pour finalement obtenir le polysaccharide dans son état de pureté maximale. À lyophiliser.

11. Utilisation du composé pharmaceutique selon la revendication 1 pour la fabrication d'un médicament pour le traitement de l'hyperplasie prostatique bénigne, dûment **caractérisée en qu'**elle consiste en l'utilisation d'un composé pharmaceutique où chaque 1 ml contient:
- Polysaccharides de Bactéries Gram négatives entre 0.05 gr & 0.002 gr.
- Thymus (extrait hydrosoluble) entre 2 mg & 0.1 mg.
- Prostate (extrait hydrosoluble) entre 1 mg & 0.01 mg.
- Total de Carbohydrates (glucose) entre 2 mg & 0.02 mg.
- Solution physiologique dépourvue de bactéries s.q.f. 1 ml.

12. Utilisation du composé pharmaceutique selon la revendication 1 pour la fabrication d'un médicament pour le traitement de l'hyperplasie prostatique bénigne conformément à la revendication 11, dûment **caractérisée en ce que** la composition contient pour chaque 100 ml.:
- Polysaccharide de Pseudomonas Aeruginosa 0.005 gr.
- Thymus (extrait hydrosoluble) 0.9 mg.
- Prostate (extrait hydrosoluble) 0.3 mg.
- Total de Carbohydrates (Glucose) 0.038 mg.
